# EUROPEAN PATENT SPECIFICATION

(11) **EP 1 982 823 B1**
(45) Date of publication and mention of the grant of the patent: **07.07.2010**
(21) Application number: 08152792.1
(22) Date of filing: 14.03.2008
(51) Int. Cl.: B29C 55/18, A61F 13/15, D06C 3/06

(54) **Process and device for treating web material**
Verfahren und Vorrichtung zur Behandlung von Bahnmaterial
Processus et dispositif de traitement de matériau de toile

(30) Priority: 19.04.2007 IT TO20070275
(43) Date of publication of application: 22.10.2008
(73) Proprietor: Fameccanica.Data S.p.A., 66020 Sambuceto di S. Giovanni Teatino (Chieti) (IT)
(72) Inventor: Pasqualoni, Paolo, 66020 Sambuceto di San Giovanni Teatino (Chieti) (IT); Lupinetti, Serafino, 65010 Elice (Pescara) (IT)
(74) Representative: Bosotti, Luciano

(56) References cited:
- WO-A-2008/012760
- DE-C- 830 413
- DE-C1- 4 342 341
- US-A- 3 624 874
- US-A1- 2003 067 091

## Description

### Field of the invention

This disclosure relates in general to techniques for treating web materials.

This disclosure has been developed with particular attention paid to its possible application to processes in which a moving web material is wound on a roller and subjected to a transverse stress, i.e., a stress exerted in the direction of the axis of the roller that would tend to displace the web material with respect to the surface of the mantle of the roller itself.

### Description of the related art

A typical context of application in which there arise conditions such as the ones delineated previously is the production of web (or sheet) materials used for producing disposable absorbent sanitary articles, said materials being designed to present characteristics of elasticity.

By "elasticity" is in general meant the fact that the material must be extendable in a clearly specified direction by a considerable amount, typically not less than 100% of its initial dimension at rest, i.e., with an elongation of 100% so that the material is brought to a length equal to twice its length at rest.

Normally, the materials to be elasticated in sanitary articles are materials made up of a number of layers, for example two layers or sheets of non-woven fabric, between which a sheet or web of elastic material is set and anchored.

To produce materials of this kind various techniques are known.

For example, there are known solutions in which the elastic material is applied between the two outer sheets that have already undergone deformation, i.e., in the state of tension. An example of this technique is, for example, represented by US-B-6 572 595.

In addition to the need to apply the elastic material that has already undergone deformation, this technique has further limitations such as the need to use sheets of non-woven fabric of greater width than that of the final elasticated sheet and the fact that the elasticity of the composite material is limited by the maximum size of the sheets of non-woven fabric.

An alternative solution (described, for example, in US-A-5 496 429, US-A-5 464 401, and US-A-5 246 433) envisages rendering elastic a composite material formed by a sheet made of elastic material sandwiched between two layers of non-woven fabric by passing it through a pair of corrugated rollers that are connected to one another. There thus occurs tearing of the fibres of the two layers of non-woven fabric, so that these no longer counter the elastic behaviour of the material set between.

This alternative solution, if compared to the solution described previously, presents certain fundamental advantages.

In the first place, it is possible to work the elastic material at the maximum of its potential.

Furthermore, it is possible to use sheets or webs of the same width as the elastic material in the resting condition.

Finally, the coupling-together of the three sheets of composite or sandwiched material does not require the use of particular equipment.

This elastication process, commonly referred to in the sector as treatment of "activation", is carried out keeping the outer edges of the sheet or web firmly in place against the stress of return exerted in the direction of its median central axis (i.e., a stress exerted in the direction of the axis of the roller that would tend to displace the web material with respect to the surface of the mantle of the roller itself) deriving from the fact that the central part passes within the grooves of the activation rollers.

It follows that the operation of activation is rendered quite complicated by the need to anchor the two side edges of the web so that the ribbed rollers can perform their action in the central portion of the web effectively.

The patent documents cited previously describe various solutions for holding the sheet in position.

For example, on the roller where the sheet or web is wound there may be present areas of openwork set at negative pressure (i.e., brought to a level of subatmospheric pressure) so as to withhold the web on the winding roller with an action of suction.

Alternatively, it is possible to use resilient elements such as disks of elastomeric material or else webs of elastomeric material that are wound on the surface of the roller.

All these solutions evidently have the purpose of withholding the side edges of the sheet or web and keeping them firmly in place in their original position, preventing sliding thereof in the direction of the axis of rotation of the roller towards the central area that is subjected to the activation treatment.

It will on the other hand be appreciated that substantially similar problems can arise also in the execution of operations of working of web materials other than the activation treatment described previously

Document EP-A-1 637 639 discloses pairs of rollers having frictional engaging means, possibly similar to sand paper, for frictionally engaging a nonwoven web, laminate or composite thereof. These rollers are used to elongate - i.e. stretching lengthwise - the web in a two-stage process, wherein the first elongation is in substantially the same direction and to substantially the same degree as the second elongation.

More specifically the invention relates to a process according to the preamble of claim 1, which is known eg. from DE 830 413 C or US 3 624 874 A1. Other documents of interest for the invention include DE 43 42 341, WO 2008/012760 A1 (which is included in the prior art under Art. 54(3) EPC) or US 2003/067091.

### Object and summary of the present invention

The object of the present invention is to provide a solution capable of exerting, during treatment of a web material that is wound on a roller, an action of lateral anchorage of at least one side of the web itself, preventing the undesired translation thereof in the direction of the axis of the roller as induced by the action of a homologous force that develops during the treatment (for example, as a result of the implementation of an activation process of the type described previously).

According to the present invention, said object is achieved thanks to a process having the characteristics recalled specifically in claim 1.

The invention relates also to a corresponding device, as called for in claim 8.

The claims form an integral part of the disclosure of the invention provided herein.

Basically, according to the embodiment described herein, the sliding of one or both of the ends of a web subjected to treatment when it is wound on a roller is prevented by providing the surface of the mantle of the roller, in an area corresponding to the area or areas where it is desired to prevent lateral displacement of the web (for example, in an area corresponding to the outer edges of the web), with a sculpturing, i.e., with a surface corrugated in such a way as to present a very high coefficient of friction.

In a particularly preferred way, the sculpturing is obtained via a treatment of diamond plating.

### Brief description of the annexed drawings

The invention will now be described, purely by way of non-limiting example, with reference to the annexed plates of drawings, in which:

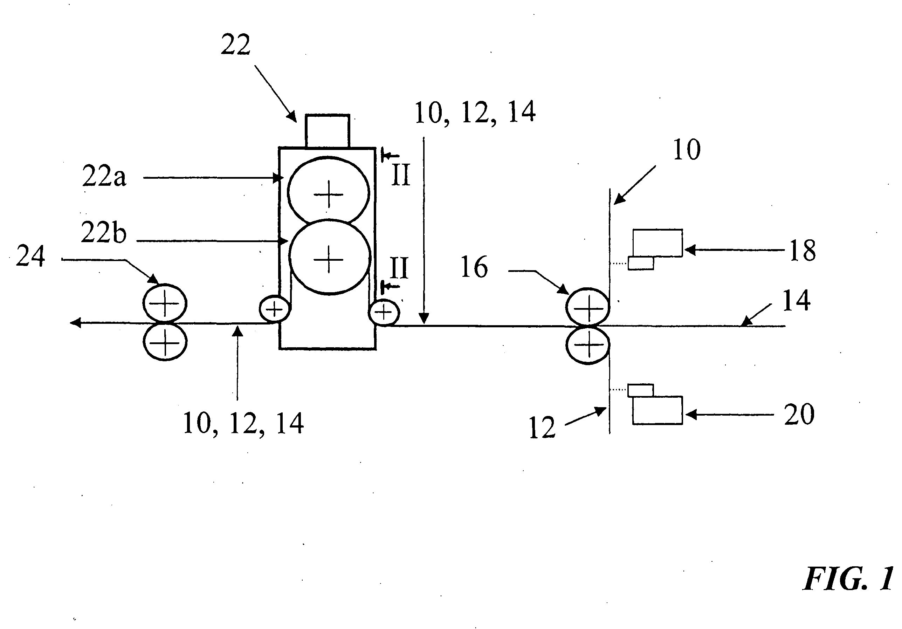

- Figure 1 is a general view in side elevation of a device operating according to the solution described herein; and
- Figure 2 corresponds by and large to a view in elevation according to the line II-II of Figure 1, reproduced at an enlarged scale.

### Detailed description of embodiments

The ensuing detailed description relates, by way of example, to the production of a web or sheet of elasticated material that can be used, for example, for the production of disposable sanitary articles.

In the specific case, the web material in question is obtained by coupling to one another two webs or sheets - the two terms are herein used in a way equivalent to one another - of non-woven fabric 10, 12, with the interposition of a web of elastic material 14.

By way of example (which of course is not to be interpreted as in any sense limiting the scope of this disclosure), the two sheets of non-woven fabric 10 and 12 can be made up of non-woven fabric with a base of polyolefin fibres, present between which are, for example, polyester or polypropylene fibres. A preferred embodiment involves the use of polypropylene fibres, with a mass per unit area in the region of 25 g/m², a thickness of 0.15 mm, and a width of 100 mm. Such a non-woven fabric is available as TB25S495DP, produced by the company Pantex Sud di Sulmona (L'Aquila - Italy).

The elastic material 14 to be sandwiched between the two sheets of non-woven fabric 10 and 12 can be constituted, for example, by an elastic material with a mass per unit area of 54 g/m², a thickness of 0.05 mm, and a width of 90 mm of the type available under the commercial name CEX 802, produced by the company Tredegar Film Products of Richmond, Virginia - U.S.A.

The sheets or webs 10, 12 and 14 (coming from respective reel sources of supply, not appearing in the drawings, but of a known type) are coupled to one another so as to produce a multilayer structure by passing it in a coupling unit 16 with counter-rotating rollers. This usually occurs after prior application of an adhesive material on the faces of the two webs of non-woven fabric 10 and 12, designed to be applied against the elastic 14.

For example, that result can be obtained using two glue-applicator units 18 and 20 of the type available under the commercial name of Summit model SME07-M2LCXE, manufactured by the company Nordson Corporation of West Lake, Ohio - U.S.A.

These glue-applicator units 18, 20 have holes for application of the glue with a diameter of 0.014 mm and a distance between centres of 6.35 mm, capable of applying the glue on the entire width of the sheets of non-woven fabric 10 and 12. Preferentially, the application of glue is a contactless application (of a spray type), which guarantees a homogeneous distribution of adhesive on the entire surface of the sheet treated. The emission of spray is guaranteed by a jet of hot air that comes out of four orifices having a diameter of 0.014 mm set on a circumference of the diameter of 2 mm having its center at the holes for exit of the glue. The air that generates the spray is supplied on the head at a pressure of 1.5 bar and is heated by the application head itself at a temperature of 145°C.

A glue that can be used in this context is the glue available under the commercial name of Dispomelt 737 from the National Starch & Chemical Company of Mezzago, Milan - Italy.

Of course, to obtain the coupling of the sheets of non-woven fabric 10, 12 and the elastic material 14 it is possible to use techniques different from the adhesive connection described. For example, it is possible for sealing to resort to mechanical-welding or ultrasound-welding systems or systems with heated rollers used individually or in combination with one another.

In the composite material 10, 12, 14 that comes out of the unit 16, the elastic material 14 is in a resting condition. In other words, the elastic web is not in a condition of tension when it is entrapped between the two sheets of non-woven fabric 10 and 12.

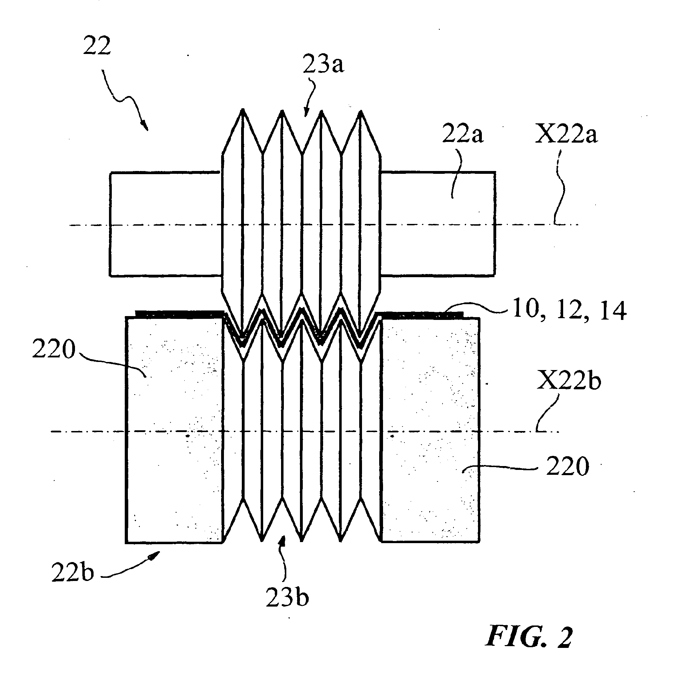

The composite material 10, 12, 14 thus obtained is then carried in the direction of an activation unit 22, substantially constituted by two counter-rotating rollers 22a and 22b having complementary ribbings 23a, 23b according to the modalities that can be more fully appreciated with reference to the view in elevation of Figure 2.

Distinguishable within the unit 22 is a "male" activator roller 22a having annular projections with a V-shaped profile (hence approximately with a general comblike configuration in the view of Figure 2) that penetrate in corresponding grooves provided in the "female" roller 22b.

In the example illustrated herein, the male roller 22a occupies a higher position than the female roller 22b. It is evident that the relative position could be reversed; or else the rollers 22a and 22b could be set alongside one another, by appropriately modifying the path of arrival of the composite web 10, 12, 14.

The composite web 10, 12, 14 is passed into the nip between the rollers 22a and 22b so as to exert the action of activation described in greater detail in the introductory part of the present description.

Basically, the activation derives from the fact that, in an area corresponding to its median central portion (see Figure 2), the composite material 10, 12, and 14 passes between the two rollers 22a, 22b and is subjected to an action that brings about weakening (in practice, tearing of the fibre) of the webs of non-woven fabric 10 and 12 as a result of the operation of tension exerted in a direction parallel to the (common) direction of the axes of rotation X22a and X22b of the two rollers 22a and 22b.

To obtain that result, the composite web 10, 12, 14 is, however, withheld in an area corresponding to its side edges, which otherwise would be simply recalled in the direction of the centre (or rather, in the direction of the longitudinal median axis) of the web, without giving rise to activation, but just to wrinkling/creasing of the central portion of the composite web.

In the solution described herein, that result is obtained by envisaging that the portions 220 set alongside one another of the skirt surface of one of the rollers 22a, 22b (of the female roller 22b, in the example illustrated herein) that occupy a lateral position with respect to the area where the activation ribbings (23a, 23b) are present, have a surface sculpturing, i.e., a general corrugated appearance represented by surface irregularities. These irregularities are adopted to get wedged between the fibres of the non-woven-fabric material of the webs 10 and 12, generating an apparent coefficient of friction that is very high, virtually tending to infinity.

This result is obtained by envisaging that the composite web 10, 12, 14 winds on one of the rollers of the activation unit 22 (once again the female roller 22b in the example illustrated herein), for a certain angular extension (typically at least equal to 90° and preferentially comprised between 90° and 180°), being kept in conditions of tension of winding on that roller.

In the exemplary embodiment illustrated herein, that result is obtained by prearranging both upstream and downstream of the activation unit 22 assemblies with counter-rotating rollers that enable the composite web 10, 12, 14 to be kept in conditions of "pull" whilst it is wound on the female roller 22a.

In the example of embodiment illustrated herein, the pair of tensioning rollers set upstream of the activation unit 22 is simply constituted by the pair of rollers of the coupling unit 16 that forms the sandwich structure of the composite web 10, 12, 14.

An as a whole homologous structure, which also comprises a pair of tensioning rollers and indicated as a whole by 24, is situated downstream of the activation unit 22.

The degree of pull (i.e., of longitudinal tension) applied on the stretch of composite web 10, 12, 14 that is wound on the roller 22b of the activation unit 22 is selectively adjustable by acting (in a known way) on the relative speed of operation of the tensioning assemblies rollers 16 and 24.

In a preferred way, the sculpturing present on the parts of the mantle 220 of the female roller 22a is a diamond plating, deriving, for example, from the application of diamond dust having dimensions of 125/149 micron, which corresponds, in the table FEPA, to a grain size of diamond DIN 151; typically deposited on the surface of the roller are 15-20 granules/mm².

Such a treatment of diamond plating can normally be implemented in the industrial field, for example at the company URMA of Turin (Italy).

As regards, instead, the extension of the winding path and the value of the longitudinal tension or pull to be applied on the web 10, 12, 14 for the desired coefficient of friction to be obtained (such as to prevent the side edges of the composite web from being recalled in the direction of the area subjected to activation) the experiments conducted by the present applicant show that an arc of winding in the region of 90° is usually amply sufficient in the presence of a value of longitudinal tension (or pull) comprised between a minimum of 6 N and a maximum of 16 N, with a preferred value of 12 N.

With these dimensional values it is possible to treat a composite web of the type described previously at a processing rate higher than 500 m/min, thus preventing any undesirable translation of the side edges of the web 10, 12, 14.

The experiments conducted by the present applicant show that the external diameter of the rollers 22a, 22b does not represent in itself a critical parameter. Purely for reasons of information, the experiments so far conducted by the present applicant in relation to a treatment of activation on the material described previously, rollers with a diameter in the region of 164 mm have enabled altogether satisfactory results to be obtained. If it is desired to render the meshing and consequent ribbing or activation of the material more gradual, it is sufficient to increase the external diameters of both of the rollers 22a, 22b. Any possible contamination with glue on the outer surfaces of the ribbings of the rollers 22a, 22b can be prevented with anti-adherent treatments or with the cooling of the rollers themselves.

Of course, without prejudice to the principle of the invention, the details of construction and the embodiments may vary widely, even to a significant extent, with respect to what is described and illustrated herein, without thereby departing from the scope of the present invention, as defined by the annexed claims.

## Claims

1. A process for subjecting a web material (10, 12, 14) to a treatment where the moving web material is wound on the skirt surface of a roller (22b) and is subjected (22) to a transverse stress exerted in the direction of the axis of said roller (22b) deriving from the fact that the central part of the web is subjected to said treatment, the process including the operations of:
- providing a surface sculpturing on at least one portion (220) of the skirt surface of said roller (22b) on which said web material (10, 12, 14) is wound by providing said surface sculpturing on two portions (220) set alongside one another of the skirt surface of said roller (22b) to co-operate with the side edges of said web material (10, 12, 14), and
- keeping under longitudinal tension said web material (10, 12, 14) that is wound on the skirt surface of said roller (22b), so that said surface sculpturing exerts on said web material (10, 12, 14) an action of anchorage countering displacement thereof induced by said transverse stress,
**characterized in that** the process includes the operation of providing said anchorage by winding said web material (10, 12, 14) on the skirt surface of said roller (22b) for an angular extension of the roller at least equal to 90°.

2. The process according to Claim 1, wherein said treatment includes the operation of subjecting said web material (10, 12, 14) to an action of weakening (23a, 23b) in said central part, said action of weakening causing on the side edges of said web material (10, 12, 14) respective transverse stresses directed in the direction of the axis of said roller (22b).

3. The process according to any one of the preceding claims, including the operation of subjecting said web material (10, 12, 14) that is wound on the surface of said roller (22b) to a force of tension between a minimum of 6 N and a maximum of 16 N, with a preferred value of 12 N.

4. The process according to any one of the preceding claims, including the operation of making said web material as a composite web (10, 12, 14) including at least one layer of elastic material (14).

5. The process according to any one of the preceding claims, including the operation of including, in said web material (10, 12, 14), at least one layer of non-woven fabric (12, 14), to co-operate with said surface sculpturing (220).

6. The process according to Claims 4 and 5, including the operation of making said web material as a sandwich structure including a layer of elastic material (14) set between two layers (10, 12) of non-woven fabric.

7. The process according to any one of the preceding claims, including the operation of providing in said web material (10, 12, 14) at least two layers (10, 14 respectively 14, 12) adhesively connected to one another.

8. A device for subjecting a web material (10, 12, 14) to treatment, including:
- a roller (22b) having a skirt surface for winding thereon said moving web material; and
- a treatment unit (22) that applies to the web that is wound on the skirt surface of the roller (22b) a transverse stress exerted in the direction of the axis of said roller (22b) deriving from the fact that the central part of the web is subjected to said treatment, the device including:
- surface sculpturing provided on at least one portion (220) of the skirt surface of said roller (22b) on which said web material (10, 12, 14) is wound wherein said surface sculpturing is provided on two portions (220) set alongside one another of the surface of said roller (22b), said two portions (220) to co-operate with the side edges of said web material (10, 12, 14); and
- tensioning elements (16, 24) for keeping under longitudinal tension said web material (10, 12, 14) wound on the surface of said roller (22b) so that said surface sculpturing exerts on said web material (10, 12, 14) an action of anchorage countering displacement thereof induced by said transverse stress,
**characterized in that**:
said tensioning elements (16, 24) keep said web material (10, 12, 14) being wound on the skirt surface of said roller (22b) for an angular extension of the roller at least equal to 90°.

9. The device according to Claim 8, wherein said treatment unit (22) is a unit for bringing about weakening of said web material (10, 12, 14) in said central part, said action of weakening causing on the side edges of said web material (10, 12, 14) respective transverse stresses exerted in the direction of the axis of said roller (22b).

10. The device according to Claim 9, wherein said roller (22b) for winding the moving web is part of said unit for bringing about weakening of said web material (10, 12, 14).

11. The device according to any one of Claims 8 to 10, wherein said surface sculpturing (220) is a diamond plating, preferably exhibiting at least one of the following characteristics:
- the diamond plating is constituted by granules of diamond with dimensions equal to 125/149 micron (grain size of diamond DIN 151, Table FEPA); and
- said granules of diamond are present in the amount of 15-20 granules/mm².

## Patentansprüche

1. Verfahren, um ein Bahnmaterial (10, 12, 14) einer Behandlung zu unterwerfen, wobei das sich bewegende Bahnmaterial auf die Manteloberfläche einer Walze (22b) gewickelt und einer transversalen Beanspruchung unterworfen wird (22), die in Richtung der Achse der Walze (22b) ausgeübt wird und sich aus der Tatsache herleitet, dass der Mittelabschnitt der Bahn der Behandlung unterworfen wird, wobei das Verfahren die folgenden Vorgänge umfasst:
- Schaffen einer Oberflächenprofilierung wenigstens auf einem Abschnitt (220) der Manteloberfläche der Walze (22b), auf die das Bahnmaterial (10, 12, 14) gewickelt wird, indem die Oberflächenprofilierung an zwei Abschnitten (220) vorgesehen wird, die längsseits der Manteloberfläche der Walze (22b) angeordnet sind, um mit den Seitenkanten des Bahnmaterials (10, 12, 14) zusammenzuwirken, und
- Halten des Bahnmaterials (10, 12, 14), das auf die Manteloberfläche der Walze (22b) gewickelt ist, unter einer longitudinalen Spannung, so dass die Oberflächenprofilierung auf das Bahnmaterial (10, 12, 14) eine Verankerungswirkung ausübt, die seiner Verlagerung, die durch die transversale Beanspruchung hervorgerufen wird, entgegenwirkt, **dadurch gekennzeichnet, dass** das Verfahren den Vorgang des Schaffens der Verankerung durch Wickeln des Bahnmaterials (10, 12, 14) auf die Manteloberfläche der Walze (22b) über eine Winkelerstreckung der Walze von wenigstens gleich 90° umfasst.

2. Verfahren nach Anspruch 1, wobei die Behandlung den Vorgang des Unterwerfens des Bahnmaterials (10, 12, 14) unter eine Schwächungseinwirkung (23a, 23b) in dem Mittelteil umfasst, wobei die Schwächungseinwirkung an den Seitenkanten des Bahnmaterials (10, 12, 14) jeweilige Querbeanspruchungen hervorruft, die in Richtung der Achse der Walze (22b) orientiert sind.

3. Verfahren nach einem der vorhergehenden Ansprüche, das den Vorgang des Unterwerfens des Bahnmaterials (10, 12, 14), das auf die Oberfläche der Walze (22b) gewickelt ist, unter eine Zugkraft zwischen mindestens 6 N und höchstens 16 N, vorzugsweise mit einem Wert von 12 N, umfasst.

4. Verfahren nach einem der vorhergehenden Ansprüche, das den Vorgang des Ausbildens des Bahnmaterials als eine Verbundstoffbahn (10, 12, 14), die wenigstens eine Lage aus elastischem Material (14) enthält, umfasst.

5. Verfahren nach einem der vorhergehenden Ansprüche, das den Vorgang des Aufnehmens wenigstens einer Lage aus Vliesmaterial (12, 14) in das Bahnmaterial (10, 12, 14), um mit der Oberflächenprofilierung (220) zusammenzuwirken, umfasst.

6. Verfahren nach den Ansprüchen 4 und 5, das den Vorgang des Ausbildens des Bahnmaterials als eine Sandwich-Struktur, die eine Lage aus elastischem Material (14) enthält, die zwischen zwei Vlieslagen (10, 12) angeordnet ist, umfasst.

7. Verfahren nach einem der vorhergehenden Ansprüche, das den Vorgang des Vorsehens von wenigstens zwei Lagen (10, 12 bzw. 14, 12) in dem Bahnmaterial (10, 12, 14), die miteinander durch Klebewirkung verbunden sind, umfasst.

8. Vorrichtung, um ein Bahnmaterial (10, 12, 14) einer Behandlung zu unterwerfen, die umfasst:
- eine Walze (22b), die ein Manteloberfläche besitzt, um das sich bewegende Bahnmaterial darauf zu wickeln; und
- eine Behandlungseinheit (22), die auf die Bahn, die auf die Manteloberfläche der Walze (22b) gewickelt ist, eine transversale Beanspruchung ausübt, die in Richtung der Achse der Walze (22b) ausgeübt wird und sich aus der Tatsache ergibt, dass der Mittelteil der Bahn der Behandlung unterworfen wird, wobei die Vorrichtung umfasst:
- eine Oberflächenprofilierung, die wenigstens in einem Abschnitt (220) der Manteloberfläche der Walze (22b) vorgesehen ist, auf die das Bahnmaterial (10, 12, 14) gewickelt wird, wobei die Oberflächenprofilierung an zwei Abschnitten (220) vorgesehen ist, die längsseits der Oberfläche der Walze (22b) angeordnet sind, wobei die zwei Abschnitte (220) mit den Seitenkanten des Bahnmaterials (10, 12, 14) zusammenwirken; und
- Spannungselemente (16, 24), um das Bahnmaterial (10, 12, 14), das auf die Oberfläche der Walze (22b) gewickelt wird, unter Spannung zu halten, so dass die Oberflächenprofilierung auf das Bahnmaterial (10, 12, 14) eine Verankerungswirkung ausübt, die seiner Verlagerung, die durch die transversale Beanspruchung erzeugt wird, entgegenwirkt,
**dadurch gekennzeichnet, dass**:
die Spannungselemente (16, 24) das Bahnmaterial (10, 12, 14) auf die Manteloberfläche der Walze (22b) über eine Winkelerstreckung der Walze von wenigstens gleich 90° gewickelt halten.

9. Vorrichtung nach Anspruch 8, wobei die Behandlungseinheit (22) eine Einheit ist, um eine Schwächung des Bahnmaterials (10, 12, 14) in dem Mittelteil hervorzurufen, wobei die Schwächungswirkung an den Seitenkanten des Bahnmaterials (10, 12, 14) jeweilige transversale Beanspruchungen hervorruft, die in Richtung der Achse der Walze (22b) ausgeübt werden.

10. Vorrichtung nach Anspruch 9, wobei die Walze (22b) zum Wickeln der sich bewegenden Bahn ein Teil der Einheit ist, um die Schwächung des Bahnmaterials (10, 12, 14) herbeizuführen.

11. Vorrichtung nach einem der Ansprüche 8 bis 10, wobei die Oberflächenprofilierung (220) eine Diamantbeschichtung ist, die vorzugsweise wenigstens eine der folgenden Eigenschaften zeigt:
- die Diamantbeschichtung ist durch Diamantkörner gebildet, deren Abmessungen gleich 125/149 Mikrometer (Korngröße von Diamant DIN 151, Tabelle FEPA) sind; und
- die Diamantkörner sind in einer Menge von 15-20 Körner/mm² vorhanden.

## Revendications

1. Procédé pour soumettre un matériau en bande continue (10, 12, 14) à un traitement où le matériau en bande continue mobile est enroulé sur la surface de jupe d'un rouleau (22b) et est soumis (22) à une contrainte transversale exercée dans la direction de l'axe dudit rouleau (22b) dérivant du fait que la partie centrale de la bande continue est soumise audit traitement, le procédé comprenant les opérations consistant à :
- fournir une sculpture de surface sur au moins une partie (220) de la surface de jupe dudit rouleau (22b) sur laquelle ledit matériau en bande continue (10, 12, 14) est enroulé en fournissant ladite sculpture de surface sur deux parties (220) placées l'une à côté de l'autre de la surface de jupe dudit rouleau (22b) pour coopérer avec les bords latéraux dudit matériau en bande continue (10, 12, 14), et
- maintenir sous tension longitudinale ledit matériau en bande continue (10, 12, 14) qui est enroulé sur la surface de jupe dudit rouleau (22b), de sorte que ladite sculpture de surface exerce sur ledit matériau en bande continue (10, 12, 14) une action d'ancrage contrant le déplacement de celui-ci produit par ladite contrainte transversale,
**caractérisé en ce que** le procédé comprend l'opération consistant à fournir ledit ancrage en enroulant ledit matériau en bande continue (10, 12, 14) sur la surface de jupe dudit rouleau (22b) pour une extension angulaire du rouleau au moins égale à 90°.

2. Procédé selon la revendication 1, dans lequel ledit traitement comprend l'opération consistant à soumettre ledit matériau en bande continue (10, 12, 14) à une action d'affaiblissement (23a, 23b) dans ladite partie centrale, ladite action de affaiblissement entraînant sur les bords latéraux dudit matériau en bande continue (10, 12, 14) des contraintes transversales respectives dirigées dans la direction de l'axe dudit rouleau (22b).

3. Procédé selon une quelconque des revendications précédentes, comprenant l'opération consistant à soumettre ledit matériau en bande continue (10, 12, 14) qui est enroulé sur la surface dudit rouleau (22b) à une force de tension entre un minimum de 6 N et un maximum de 16 N, avec une valeur préférée de 12 N.

4. Procédé selon une quelconque des revendications précédentes, comprenant l'opération consistant à réaliser ledit matériau en bande continue sous forme de bande continue composite (10, 12, 14) comprenant au moins une couche de matériau élastique (14).

5. Procédé selon une quelconque des revendications précédentes, comprenant l'opération consistant à inclure, dans ledit matériau en bande continue (10, 12, 14), au moins une couche de tissu non tissé (12, 14), pour coopérer avec ladite sculpture de surface (220).

6. Procédé selon les revendications 4 et 5, comprenant l'opération consistant à réaliser ledit matériau en bande continue sous forme de structure sandwich comprenant une couche de matériau élastique (14) placée entre deux couches (10, 12) de tissu non tissé.

7. Procédé selon une quelconque des revendications précédentes, comprenant l'opération consistant à fournir, dans ledit matériau en bande continue (10, 12, 14), au moins deux couches (10, 14, respectivement 14, 12) reliées l'une à l'autre de façon adhésive.

8. Dispositif pour soumettre un matériau en bande continue (10, 12, 14) à un traitement, comprenant :
un rouleau (22b) comportant une surface de jupe, pour enrouler sur celle-ci ledit matériau en bande continue mobile ; et
une unité de traitement (22) qui applique sur la bande continue, qui est enroulée sur la surface de jupe du rouleau (22b), une contrainte transversale exercée dans la direction de l'axe dudit rouleau (22b) dérivant du fait que la partie centrale de la bande continue est soumise audit traitement, le dispositif comprenant :
- une sculpture de surface prévue sur au moins une partie (220) de la surface de jupe dudit rouleau (22b), sur laquelle ledit matériau en bande continue (10, 12, 14) est enroulé, dans lequel ladite sculpture de surface est prévue sur deux parties (220) placées l'une à côté de l'autre de la surface dudit rouleau (22b), lesdites deux parties (220) étant destinées à coopérer avec les bords latéraux dudit matériau en bande continue (10, 12, 14) ; et
- des éléments tendeurs (16, 24) pour maintenir sous tension longitudinale ledit matériau en bande continue (10, 12, 14) enroulé sur la surface dudit rouleau (22b) de sorte que ladite sculpture de surface exerce sur ledit matériau en bande continue (10, 12, 14) une action d'ancrage contrant le déplacement de celui-ci produit par ladite contrainte transversale,
**caractérisé en ce que** :
lesdits éléments tendeurs (16, 24) maintiennent ledit matériau en bande continue (10, 12, 14) enroulé sur la surface de jupe dudit rouleau (22b) pour une extension angulaire du rouleau au moins égale à 90°.

9. Dispositif selon la revendication 8, dans lequel ladite unité de traitement (22) est une unité pour entraîner un affaiblissement dudit matériau en bande continue (10, 12, 14) dans ladite partie centrale, ladite action d'affaiblissement entraînant, sur les bords latéraux dudit matériau en bande continue (10, 12, 14), des contraintes transversales respectives exercée dans la direction de l'axe dudit rouleau (22b).

10. Dispositif selon la revendication 9, dans lequel ledit rouleau (22b) pour enrouler la bande continue mobile fait partie de ladite unité pour entraîner l'affaiblissement dudit matériau en bande continue (10, 12, 14).

11. Dispositif selon une quelconque des revendications 8 à 10, dans lequel ladite sculpture de surface (220) est un placage diamant, présentant de préférence au moins une des caractéristiques suivantes :
- le placage diamant est constitué par des granules de diamant avec des dimensions égales à 125/149 microns (grosseur de grain de diamant DIN 151, Table FEPA) ; et
- lesdits granules de diamant sont présents dans la quantité de 15 à 20 granules/mm².
